# EUROPEAN PATENT SPECIFICATION

(11) **EP 2 695 607 B1**
(45) Date of publication and mention of the grant of the patent: **04.03.2020**
(21) Application number: 12768480.1
(22) Date of filing: 04.04.2012
(51) Int. Cl.: A61K 9/127, A61K 38/16, A61K 47/18, A61K 47/24, A61K 47/28, A61K 47/44

(54) **METHOD FOR PRODUCING ENDOPLASMIC RETICULUM**
VERFAHREN ZUR HERSTELLUNG EINES ENDOPLASMATISCHEN RETIKULUMS
PROCÉDÉ DE PRÉPARATION DE RÉTICULUM ENDOPLASMIQUE

(30) Priority: 04.04.2011 US 201161471490 P
(43) Date of publication of application: 12.02.2014
(73) Proprietor: Waseda University, Tokyo 169-8050 (JP)
(72) Inventor: SAKAI, Hiromi, Tokyo 169-8050 (JP)
(74) Representative: Nordmeyer, Philipp Werner
(86) International application number: PCT/JP2012/059233
(87) International publication number: WO 2012/137834

(56) References cited:
- EP-A1- 0 687 463
- JP-A- 3 173 813
- JP-A- 4 256 430
- JP-A- 6 071 110
- JP-A- 63 209 746
- JP-A- 2003 290 642
- JP-A- 2009 035 517
- US-A- 4 776 991
- US-A- 4 935 171
- US-A1- 2004 247 660
- US-A1- 2011 024 929
- US-A1- 2011 059 157

## Description

### Field of the Invention

The present invention relates to a method for producing a phospholipid vesicle (liposome) encapsulating hemoglobin, suitable as an artificial oxygen carrier (hemoglobin vesicle) with greater efficiency than had been conventionally possible.

### Description of the Related Art

The current system for blood donation and transfusion has been established as an indispensable technique for clinical medicine. With many problems (infection, shelf life, decrease in the number of blood donors, and so on), however, it is an urgent challenge to find an alternative to the donated blood. Especially needed is the development of an alternative to red blood cells, which have an oxygen-carrying function. As hemoglobin (Hb), which is contained in red blood cells at a high concentration, is an oxygen-binding protein, an artificial red blood cell using hemoglobin has been developed. It is possible to completely remove pathogens and blood group antigens from hemoglobin by rigorous purification from red blood cells. Versions of chemically modified hemoglobins (those bound to water-soluble polymers, those with intramolecular crosslinks, and those which are polymerized) as well as hemoglobin vesicles and the like which encapsulate highly purified hemoglobin in the inner aqueous phase of phospholipid vesicles or polymer capsules have been known as artificial oxygen carriers that utilize hemoglobin. Reviewing global trends, the development of modified hemoglobin solutions took a lead, and some of them reached the final phase of clinical trials. Series of development projects have been abandoned due to unexpected adverse effects and selection is still in progress. This is because they lack a physiologically significant structure of the red blood cell. The red blood cell is a discoid and biconcave particle of approximately 8 micrometers in diameter with a depressions at the center thereof, which has highly concentrated (about 35%) hemoglobin (molecular weight: 64,500), which is toxic in nature, encapsulated within the red blood cell membrane. Chemical modification of hemoglobin alone cannot eliminate hemoglobin toxicity completely (Non-patent Document 1; Sakai H. and Tsuchida H. (2009) Pharmacia, 45:23-28 (in Japanese)).

Historically, it was reported in the late 1960's that self-assembly of an amphiphilic molecule, phospholipid, in water results in a bilayer membrane, which composes a vesicle structure (phospholipid vesicle, liposome). Since the late 1970's, attempts have been made to encapsulate hemoglobin in the phospholipid vesicle. Such encapsulation was not realized, however, because preparation was difficult such as control of vesicle size, and because there were not sufficient means to prevent aggregation caused by interaction with blood plasma proteins (Non-patent Document 2; Djordjevich L. and Miller I.F. (1977) Fed. Proc., 36: 567). Later, however, a technique to cover a highly concentrated solution of hemoglobin with phospholipid bilayer membrane employing an extrusion method, and control of vesicle size such that vesicles could flow, especially through blood capillaries, as well as improvements in dispersion stability were achieved, and a hemoglobin vesicle was reported as being produced (Non-patent Document 3; Sakai H, Hamada K, Takeoka S, Nishide H, Tsuchida E. (1996) Biotechnol. Prog., 12: 119-125). US patent application US 2011/0059157 A1 discloses encapsulation materials, in particular, compositions comprising anionic lipids without a phosphate group. US patent No. 4,776,991 relates to a method of making liposome-encapsulated hemoglobin with the liposome made from chloroform, HSPC, cholesterol, negatively charged DMPG and alpha-tocopherol. It was possible to suppress aggregation among vesicular particles and to increase the dispersion stability by disposing polyethylene glycol on particle surfaces. It was also possible to preserve the vesicle in a liquid state for a long period of time at room temperature by removing oxygen completely from the suspension (Patent Document 1; Japanese Patent No.: 3466516). Animal testing were conducted and the safety of the hemoglobin vesicle as an alternative to blood transfusion and the details of oxygen-carrier functions were clarified (Non-patent Document 4; Sakai H, Sou K, Horinouchi H, Kobayashi K, Tsuchida E. (2008) J. Intern. Med., 263: 4-15). So far, its use as a resuscitation fluid for hemorrhagic shock, as a supplementary administration (dilution of blood) to patients with repetitive bleedings during surgical operations, as a prime fluid for extracorporeal circulation circuits (artificial heart-lung machines) and the like have been examined and the hemoglobin vesicle has been demonstrated to have a sufficient efficacy as an oxygen carrier. For diseases which cannot be treated by transfusions, prevention of expansion of infarct zone by administering to models of cardiac and cerebral infarction, supply of oxygen in ischemic region of pedicled flap, improvement of the efficacy of radiation therapy by oxygenation, and use as an oxygen source for an organ to be transplanted and for cultured cells, have been examined (Non-patent Document 5; Tsuchida E, Sou K, Nakagawa A, Sakai H, Komatsu T, Kobayashi K. (2009) Bioconjug. Chem., 20: 1419-40).

### Disclosure of the Invention

Known methods for preparing phospholipid vesicles include ultrasonic irradiation, reverse-phase preparation with organic solvents, dispersion with a surfactant followed by subsequent removal with dialysis, and the like (Non-patent Document 6; *"*Liposomes," ed. by Nojima S., Sunamoto J., Inoue K. (1988) Nankodo, Tokyo Japan). For the manufacture of a preparation comprising a functional protein such as hemoglobin of which administration into blood vessels is presumed, however, these methods are not suitable because of concerns regarding protein denaturation during the manufacturing process and regarding the presence of residual substances. In addition, in view of the large dose to be administered compared to liposome preparations in general, these methods are extremely inefficient in producing an artificial red blood cell preparation. The ratio of hemoglobin weight per unit lipid weight is employed as a parameter representing the performance of an individual particle of the artificial red blood cell. The higher the value of the parameter, the more efficiently the hemoglobin-bound oxygen can be delivered. To this effect, it is necessary to set the hemoglobin concentration in the inner aqueous phase of the particle as high as possible. More specifically, it is required to disperse complex lipid in a solution of highly concentrated hemoglobin (e.g. 35-45g/dL) and to encapsulate the highly concentrated hemoglobin while the vesicles are formed. The solution of highly concentrated hemoglobin is viscous and as lipid powder is dispersed in it, it will become more viscous.

In case the particle size is controlled according to the so-called Extrusion Method by passing through filters with different pore sizes in a stepwise manner (for example, passing through MF filters of EMD Millipore Corporation with pore sizes of 3.0 micrometers, 0.8 micrometers, 0.6 micrometers, 0.45 micrometers, 0.3 micrometers, and 0.22 micrometers in this order), changing the filters is tedious and the filters tend to clog. To circumvent these problems, it has been known to use freeze-dried powder of vesicles which have been formed with the lipid in the aqueous solution (Non-patent Document 7; Sou K., Naito Y., Endo T., Takeoka S., Tsuchida E. (2003) Biotechnol. Prog., 19: 1547-1552; Patent Document 2; Japanese Patent Application No. 2000-344459 (WO2002/038128)). However, removing water by freeze drying takes an extremely long time and costs are high. Thus, with industrial implementation in mind, the low efficiency has been a problem. Furthermore, dried vesicles of small particle sizes may be included and, after dispersion in the hemoglobin solution, may still persist without sufficiently encapsulating hemoglobin. Considering the efficiency of mixing and extrusion, there was a limitation in the weight of dry lipid that can be supplemented in the viscous and dense solution of hemoglobin. The upper limit was 6 g/dL at most (meaning that 6 g of lipid is dispersed in 1 dL of dense hemoglobin solution). There were also problems that, after the mixing, it takes a long time to remove a large amount of foam which is formed, that the foam contributes to denature proteins, and that the lipid powder remains as lumps without being dispersed completely.

As a method for dispersing dried powder of complex lipid into the viscous and dense solution of hemoglobin using a blade agitator, it has been an additional problem that it takes a long time to implement the method because lipid lumps may sometimes be formed, that the foam formed during hydration of the lipid powder hardly disappears in the viscous solution, reducing the filtration rate in the extrusion procedure, and that the lipid lumps, which could not be dispersed, remain on the filter and result in loss. Recovery yield for the hemoglobin was 20% at best. The hemoglobin which was not included in the vesicles had to be recycled by recollection and re-concentration, or had to be discarded. Thus, this method was extremely inefficient.

Another known method comprises a step of reducing particle size by generating shear stress by causing collision of opposing flows of viscous solutions of hemoglobin dispersed with complex lipid at high velocity and pressure (Non-patent Document 8; Beissinger R.L., Farmer M.C., Gossage J.L. (1986) ASAIO Trans., 32: 58-63). In this method, however, it was difficult to control the shear stress. In order to pass the solutions of hemoglobin dispersed with lipid through a circuit, the solutions were required to have some degree of fluidity, and therefore, the lipid concentration had to be reduced to about 6 g/dL. As a result, the recovery yield of hemoglobin was as low as about 20%.

Still another known method comprises the steps of forming a paste by emulsifying lipid powder with a small volume of water to become hydrated and swell, and encapsulating hemoglobin by mixing the paste with a solution of hemoglobin at a high speed to form an emulsion (Patent Document 3; Japanese Published Patent Application No.: 2009-035517). When the dried lipid was hydrated with the small volume of water, it was possible that vesicles were formed. After mixing with the hemoglobin, the vesicles may remain empty (only water inside) without encapsulating the hemoglobin. As a result, the hemoglobin could not be encapsulated efficiently. Thus, reduced encapsulation efficiency is expected.

The present invention was developed in view of the above-mentioned problems. An object of the present invention is to provide a novel method as claimed, for producing a preparation of phospholipid vesicles (liposomes).

Another object of the present invention is to provide a method for producing efficiently a dense solution of a dispersed artificial oxygen carrier (hemoglobin vesicle) which is used, especially in the medical field, as an alternative to blood transfusions.

A further object of the present invention is to provide a method for producing a preparation of phospholipid vesicles (liposomes) which increase the recovery yield of hemoglobin remarkably better than had been conventionally possible, with more simplified processes, shorter operation time, and higher biocompatibility.

In order to enhance oxygen carrier performance of an individual particle, it is also important to mix dried complex lipid powder directly with a dense hemoglobin solution. Thus it is also an object of the present invention to provide an efficient method for mixing "a large amount of bulky powder of dried complex lipid" with a "viscous and dense solution of hemoglobin," encapsulating the dense solution of hemoglobin inside the vesicles, controlling the particle size and increasing the recovery yield of hemoglobin.

The phospholipid vesicle first contacts blood components after administration into a blood vessel. It has been known that serious complement activations are triggered depending on the lipid composition of the phospholipid vesicle, affecting hemodynamics (Non-patent Document 9; Szebeni J. (2005) Toxicology, 216: 106-121). It has been understood that a small amount of negatively-charged lipid is effective in encapsulating hemoglobin into phospholipid vesicles efficiently. It was reported, however, that some of the negatively-charged lipids can trigger a complement activation and that whether the complement reaction is triggered depends on the molecular species of the negatively-charged lipids. Therefore, it is important to select a particular species of the negatively-charged lipids. In addition, as the major lipid component of phosphatidylcholine-type phospholipids, 1, 2-dipalmitoyl-sn-glycero-3-phosphoatidylcholine (DPPC) and hydrogenated lecithin derived from soybeans (HSPC) are frequently used. Their gel-liquid phase transition temperatures are as high as 41°C and 50-56°C, respectively. During the course of completion of the present invention, it was realized that higher energy (shear stress, agitation speed) is required to control the particle size by dispersing in the dense hemoglobin solution complex lipid comprising lipid with a higher phase transition temperature. It was required to select a lipid with a lower phase transition temperature (below 30°C). The lipid membrane composition of phospholipid vesicles is heterogeneous with multiple components namely: phosphatidylcholine-type phospholipid, cholesterol, a negatively-charged lipid, and a lipid bound with polyethylene glycol. Thus, it is an object of the present invention to select each of the components and to determine their compound ratio.

Considering the above-mentioned background and problems and studying diligently, the inventors of the present invention have conceived of a method for preparing an artificial red blood cell by adopting the principle of kneading operations, to disperse dried lipid powder homogeneously in a dense hemoglobin solution, to form a small vesicle, to control the particle size, to increase recovery yield of hemoglobin, and to suppress denaturation of hemoglobin during the operation. For example, although a mixer/deaerator (Patent Document 4; Japanese Patent No.: 3860770; Patent Document 5; Japanese Patent No.:2711964) is being used to prepare a kneaded material (a paste-like material in which a small amount of liquid is mixed with a large amount of powder), the present inventors discovered that the mixer/deaerator has a function other than simple mixing. The present inventors also focused on the point that the machine can carry out the operation efficiently. Basically, the machine carries out planetary movement of simultaneously revolving while rotating a cylindrical container in which a sample to be kneaded is included, so that the sample can be homogeneously mixed and foam can be destroyed by the centrifugal force of revolution. By kneading in a closed container, it is possible to maintain a sterile atmosphere. In one embodiment of the present invention, a larger amount of lipid powder than before can be added and dispersed in a hemoglobin solution, and a solution of dispersed hemoglobin vesicle can be prepared efficiently. In addition, it is possible not only to disperse the lipid but also to control the particle size of the vesicles which are formed by the self-assembly of lipid molecules. As a strong shear stress is generated, it is important to pretreat the hemoglobin to be bound to carbon monoxide or to be transformed as deoxyhemoglobin in order to stabilize the hemoglobin at divalent iron state. In the prior art, it has been known that binding of carbon monoxide to hemoglobin enables viral inactivation by heating treatment (low temperature pasteurization) during the purification process of hemoglobin (Patent Document 6; Japanese Patent No.: 3331433), that a transfusion preparation has a cytoprotective effect by administering hemoglobin vesicle which is bound to carbon monoxide (Patent Document 7; Japanese Published Patent Application No.:2007-269665), and that a transfusion preparation can be stored for a long time by transforming to deoxyhemoglobin (Patent Document 8; Japanese Patent No.: 3466516). The present invention is the first to use this technique to prevent denaturation during the kneading operation.

Conventionally, examples of application of kneading machines (mixer/deaerator) include: gold/silver/carbon paste and the like for electrical conducting/resistor materials; glass/ceramic paste and the like for sealing/insulating materials; epoxy resin and the like for sealing materials/adhesives; LED fluorescent agent silicone resin and the like for molding materials; ink/paint/pigment and the like for mixing different coloring materials; dental materials/pharmaceutical ointment materials/cosmetic base materials and the like for manufacturing; precision parts and the like for abrasive purposes; and for emulsifying oil and water such as in mayonnaise which are immiscible in nature, in the food industry. There has been no example, like the present invention, of dispersing lipid molecules to form molecular assembly or using a kneading machine to prepare a liposome for i.v. injection. The reason is presumably because, in the conventional so-called liposome preparation, there has been no need to encapsulate such a high concentration of solution, or at such a high efficiency, or to administer such a high dose. It was first reported in 1977 (Non-patent Document 2; Djordjevich L. and Miller I.F. (1977) Fed. Proc., 36: 567) that hemoglobin is encapsulated in phospholipid vesicles (liposomes). Thirty-four years have passed since then. Development of kneading machines based on the planetary movement of containers dates back to earlier than 1992 (Patent Document 5; Japanese Patent No.: 2711964), from which nineteen years have passed. During this period, as there has been no example of the kneading technique being used for the preparation of hemoglobin vesicles, it is apparent that the present invention could not be readily conceived of by those skilled in the art.

Besides the above-mentioned mixer/deaerator apparatus (e.g. manufactured by THINKY CORPORATION, KURABO INDUSTRIES LTD., etc.), apparatuses are known that have an kneading effect by a rotation and revolution movement (planetary movement) with two twisted open frame type blades as well as an action of impact and shear by a turbine blade (PD Mixer, Planetary Mixer, etc. manufactured by INOUE MFG., INC.), thus these apparatuses may be used as well with an effort to control the pressure for destroying the foam, an effort to suppress foaming, and an effort to generate enough shear stress.

As for the phosphatidylcholine-type phospholipids, which are the major component, it is easy to disperse complex lipid powder, to promote the forming of vesicles, and to control particle size by using one which has the lowest possible phase transition temperature. Conventionally, a negatively charged lipid had been required in order to increase encapsulation efficiency. However, it is possible to increase the biocompatibility with 1,5-O-dihexadecyl-N-succinyl-L-glutamate (Non-patent Document 10; Sou K., Tsuchida E. (2008) Biochim. Biophys. Acta, 1778: 1035-1041). As such, it is extremely critical to select the components and composition of the phospholipid vesicle as multicomponent complex.

In case the particle size of the hemoglobin vesicle is about 200 - 300 nm, it may be necessary to apply some pressure for the hemoglobin vesicle to pass through a sterile filter with a pore size of 0.22 micrometers, so that the sterility could not be guaranteed. In such a case, beta-propiolactone is to be supplemented in the final step of the process (Non-patent Document 11; LoGrippo G.A., Wolfran B.R., Rupe C.E. (1964) J.A.M.A., 187: 722-766). As this reagent may induce protein denaturation according to the principle of sterility, it is important to stabilize hemoglobin beforehand by the above-mentioned methods. As beta-propiolactone is carcinogenic in nature, it is necessary to confirm that the residual compound is completely inactivated by hydrolysis.

To achieve the above-mentioned objects, the claimed method is provided for producing vesicles encapsulating a functional substance comprising the steps of: (a) containing the functional substance, lipid and water in a cylindrical container; and (b) producing lipid vesicles which comprise the lipid as a main component and which encapsulate the functional substance therein, by kneading the contents of the cylindrical container with simultaneous movements of rotating the cylindrical container around its center axis together with revolving the cylindrical container about a predetermined axis of revolution.

The claimed method is provided for producing vesicles, wherein the contents contained in the cylindrical container in step (a) of the above-mentioned method are prepared by adding the lipid in an aqueous solution comprising hemoglobin. Furthermore, a method is provided for producing vesicles, wherein the content contained in the cylindrical container in step (a) of the above-mentioned method is prepared by dispersing a lipid powder in the water, the lipid powder being obtained by drying the lipid comprising hemoglobin. Also, a method is provided for producing vesicles, wherein the functional substance is hemoglobin and the aqueous solution of step (a) is prepared by adding 15g or more of a complex lipid powder as the lipid per 1 dL of an aqueous solution of hemoglobin in which 30 - 50 g/dL of hemoglobin is dissolved. Also, a method is provided for producing vesicles, wherein in step (b) of the above-mentioned method the aqueous solution is kneaded by revolving and rotating the cylindrical container at a rate of revolution for the cylindrical container of 200 - 300 rpm and at a rate of rotation for the cylindrical container of 100 - 3000 rpm. Also, a method is provided for producing vesicles further comprising the steps of: (c), after step (b), adding water or saline to a liquid or paste in the cylindrical container; and (d), after step (c), reducing the viscosity of the liquid or paste in the cylindrical container by further rotating the cylindrical container around the center axis together with revolving the cylindrical container about the predetermined axis of revolution. Also, a method is provided for producing vesicles further comprising a step (e), after step (d), removing the functional substance which is not encapsulate with the lipid by applying an ultrafiltration membrane technique or ultracentrifugation technique to the fluid or paste in the cylindrical container.

In addition, according to an embodiment of the present invention, a method is provided for producing vesicles, wherein in step (b) of the above-mentioned method, the step of kneading the aqueous solution by rotating the cylindrical container around the center axis while the cylindrical container revolves about the predetermined revolution axis is implemented multiple times, and during the interval between two consecutive kneading steps, a cooling treatment is performed to cool down the fluid or paste by stopping at least one of rotation and revolution of the cylindrical container or by reducing the rate of at least one of rotation and revolution of the cylindrical container. Also, a method is provided for producing vesicles, wherein the cylindrical container of the above-mentioned method has multiple concave-curved surfaces on the inner periphery of its sidewall and the centers of curvature of the adjacent concave-curved surfaces are at different positions, whereby a convex-shaped crest that protrudes toward the interior of the cylindrical container is formed between the adjacent concave-curved surfaces.

In addition, according to an embodiment of the present invention, a method is provided for producing vesicles, wherein the hemoglobin is carbonyl hemoglobin with its heme at a divalent iron state or deoxyhemoglobin with its heme at a divalent iron state. Also, a method is provided for producing vesicles wherein the functional substance is hemoglobin and the aqueous solution of steps (a) is prepared by adding 15 g or more of a complex lipid per 1 dL of an aqueous solution of hemoglobin in which 30 - 50 g/dL of hemoglobin is dissolved; and further comprising a step of removing contaminating unstable proteins by denaturation with a treatment that heats the aqueous solution of hemoglobin to 50 °C or higher for five hours or longer before adding the lipid to the aqueous solution of hemoglobin, and removing the contaminating unstable proteins with an ultrafiltration membrane or centrifugation, wherein the removing step is implemented in order to reduce the occurrence of insoluble matter of the denatured protein in the course of the kneading treatment in step (b). The claimed method is provided for producing vesicles, wherein the lipid is comprised of a phosphatidylcholine-type phospholipid of 1, 2-dipalmitoyl-sn-glycero-3-phosphatidylcholine, cholesterol, a negatively-charged lipid of 1, 5-O-dihexadecyl-N-succinyl-glutamate, and a lipid bound with polyethylene glycol of 1, 2-distearoyl-sn-glycero-3-phosphatidylethanolamine-N-poly(oxyethylene)5000 (molecular weight of the polyethylene glycol chain: 5,000). Also, a method is provided for producing vesicles, wherein the gel-liquid phase transition temperature of the phosphatidylcholine-type phospholipid is 30°C or lower and below that of 1,2-dipalmitoyl-sn-glycero-3-phosphatidylcholine.

Also, a method is provided for producing vesicles, comprising a step of producing dried lipid powder from a lipid lamellar gel as the lipid of the above-mentioned method, wherein the step of producing the dried lipid powder comprises steps of producing an aqueous solution of the lipid by adding 15 g/dL or more of the lipid powder in pure water which is comprised of substantially no solute; kneading the aqueous solution of lipid within a cylindrical container, rotating the cylindrical container around its center axis together with revolving the cylindrical container about a predetermined axis of revolution, and obtaining the dried lipid powder from the lipid lamellar gel by freeze-drying the kneaded aqueous solution of lipid. In the above method the lipid molecules spontaneously self-assemble to compose the vesicle and simultaneously to encapsulate the functional substance, and the particle size of the vesicle is controlled by shear stress generated by the kneading.

In addition, according to an embodiment of the present invention, a method is provided for producing hemoglobin vesicles, wherein the lipid composition of the dried powder of the complex lipid in the claimed method is composed of a phosphatidylcholine-type phospholipid, cholesterol a negatively-charged lipid and a lipid bound with polyethylene glycol, wherein the gel-liquid phase transition temperature of the phosphatidylcholine-type phospholipid is below that of 1,2-dipalmitoyl-sn-glycero-3-phosphatidylcholine.

According to the present invention, hemoglobin vesicles can be produced in a shorter time with higher yield than had been conventionally possible. It is shown that the operation of the present invention not only kneads lipid powder and dense hemoglobin solution homogeneously, but also disperses lipid molecules in the hemoglobin solution, encapsulates hemoglobin in the inner aqueous phase of the higher order structure of the lipid bilayer membrane (liposome capsule) which is spontaneously reorganized, and is capable of controlling the particle size of the hemoglobin vesicle at an optimum value. Thus, the present invention enables greater simplification of operation processes than expected as a whole. In addition, it is possible to increase production efficiency in a method for encapsulating a functional protein other than hemoglobin and low molecular weight compounds, a method for producing vesicles, and a method for producing a lipid gel.

The features of the present invention are defined in the claims. Remarkable actions and effects other than those described above will be apparent to those skilled in the art by referring to the following description of embodiments and figures that exemplify the principle of the present invention.

### BRIEF DESCRIPTION OF THE DRAWINGS

Fig. 1 is a schematic representation showing a kneading machine of one embodiment of the present invention.
Fig. 2 is a magnified schematic representation showing a part of the kneading machine.
Fig. 3 is a cross-section view of an exemplary cylindrical container.
Fig. 4 is a cross-section view of an exemplary cylindrical container.

### BEST MODE FOR CARRYING OUT THE INVENTION

Embodiments of the present invention will be described below.

Hemoglobin vesicles of the present embodiment comprise phospholipid vesicles (liposomes) composed of lipid bilayer membranes, of which phosphatidylcholine-type phospholipid, cholesterol, a negatively-charged lipid, and a lipid bound with polyethylene glycol, and in which hemoglobin is included. The hemoglobin vesicle of this embodiment also comprises a capsule formed by a surfactant which is co-polymerized with a hydrophilic polymer and a hydrophobic polymer (polymersome) in which the hemoglobin is included. The hemoglobin solution may be derived from donated human blood, domestic animals, or recombinant hemoglobin. Although hemoglobin, a globular protein, is relatively stable among proteins, oxyhemoglobin will be converted into methemoglobin by auto-oxidation over time. Heat may be generated by shear stress during kneading with a kneading machine of this embodiment (mixer/deaerator: an apparatus which rotates and revolves at the same time). It is better to pretreat the hemoglobin with carbon monoxide to convert the hemoglobin to carbonyl hemoglobin (HbCO), or to remove oxygen completely to convert the hemoglobin to deoxyhemoglobin (deoxyHb), in order to give heat tolerance to the hemoglobin with the center iron of the heme in a ferrous state. By keeping the hemoglobin concentration as high as 35-45g/dL, the concentration of hemoglobin encapsulated in the vesicle may also be kept high. A dense solution of hemoglobin, the raw material, is concentrated following repeated dialysis with ultrafiltration membrane, and it is necessary to prevent clogging of the ultrafiltration membrane. It may be difficult to separate contaminated proteins which are prone to denaturation (residual glycolytic enzymes, enzymes for reducing metHb, carbonic anhydrase and the like) as they come to be denaturated and insoluble during the course of kneading. It is, therefore, preferable to use hemoglobin which has been heat treated (at 60 °C, for 10 - 12 hours) to remove the denatured contaminated proteins. The hemoglobin may be supplemented with a previously determined amount of pyridoxal 5'-phosphate or chlorine ions as an allosteric effector. For targeting oxygen to an ischemic region, the hemoglobin may also be encapsulated in liposome without supplementing any allosteric effectors.

The weight ratio of hemoglobin to lipid is a parameter representing the oxygen carrier efficiency of hemoglobin vesicle. Supposing that the ratio is about 2.0 and recovery yield is aimed at 100%, for example, it is a guide to supplement with 20 g of lipid powder, as 40 g of hemoglobin is resolved in 1dL of hemoglobin solution at 40 g/dL. According to our study, good results are obtained when 15 g or more (preferably 20 - 25 g) of dried lipid powder is supplemented to 1dL of dense solution of hemoglobin at 35 - 45 g/dL, and the mixture is kneaded.

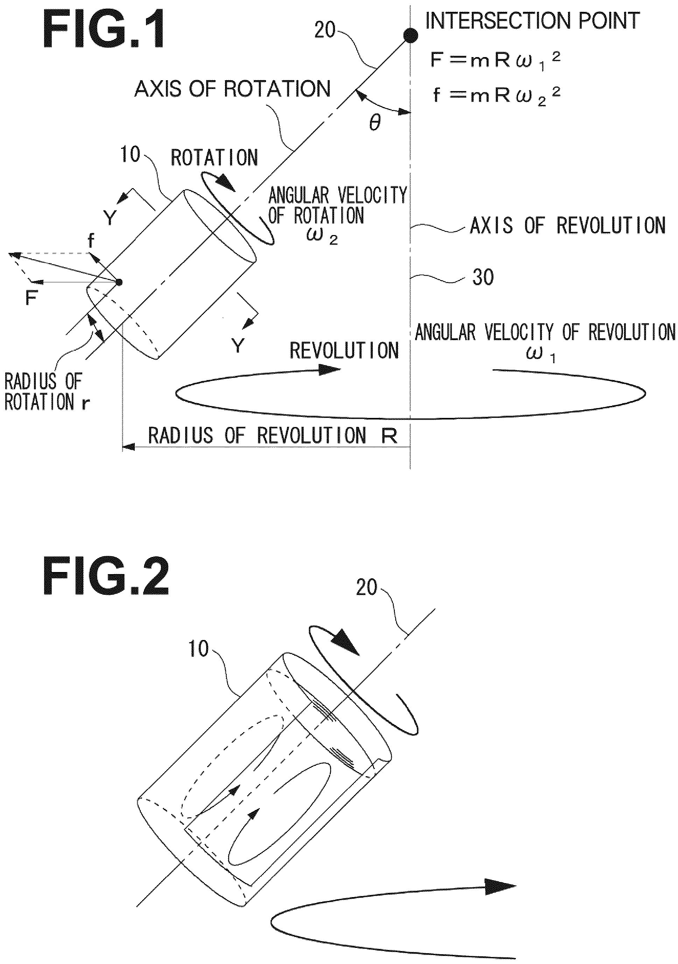

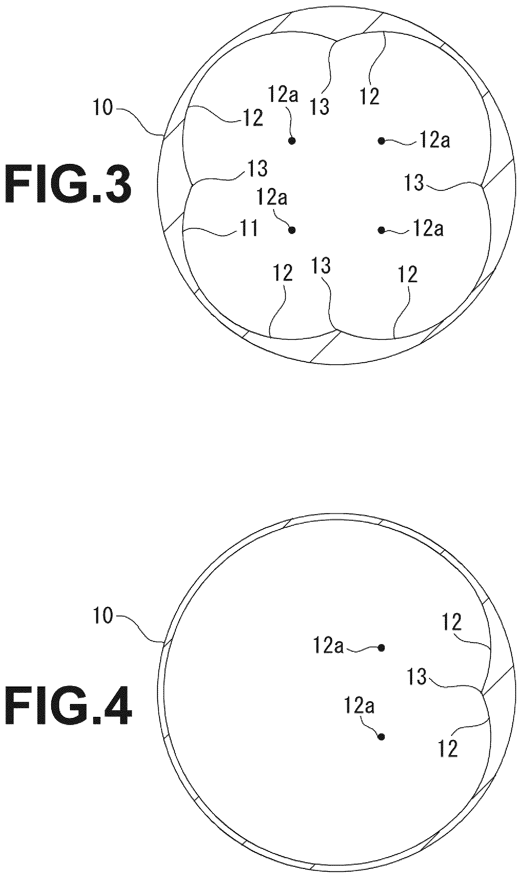

As a method for kneading, it is good to use a kneading machine based on planetary movement with revolution and rotation. For example, a kneading procedure (revolution: 500 -1500 rpm; rotation: 200 - 600 rpm) for 3 - 6 minutes while the mixture is sealed in a sterile cylindrical container made of stainless steel, polyethylene, Teflon (registered mark) and the like, is repeated 30 times using a kneading machine manufactured by THINKY CORPORATION (mixer/deaerator. ARE-310). As shown in the schematic representations of Figs. 1 and 2, the kneading machine has a cylindrical container 10 with a structure in which the cylindrical container 10 rotates around a center axis 20 (axis of rotation) and revolves about a center axis 30 (axis of revolution) at the same time. The axis of rotation 20 is not on parallel with the axis of revolution 30. Preferably the axis of rotation 20 is at an angle of θ with respect to the axis of revolution 30. Preferably θ is 30° or more and 60° or less. In each of the examples described below, the axis of rotation 20 is at an angle of about 45°, a median value of the above-mentioned angle range, with respect to the axis of revolution 30. By setting the angular orientation in this manner, good results were obtained as will be described below. In addition, in case the angle of the axis of rotation 20 with respect to the axis of revolution 30 is set in the above-mentioned range, results are obtained which are similar to those described below. Therefore, as for the shape of the cylindrical container and kneading conditions, it is preferable but not limited to set the angle θ within the above range of angles. The angle value θ may be set at an angle near 0° or over 60°, for example, using a cylindrical container with barrel-shaped bulging sides. Thus, it is possible, and the present invention does not exclude, setting the angle value θ out of the above-mentioned range of angles. It is possible to promote the kneading by using a cylindrical container which does not have a smooth inner wall but an inner wall with an obstacle on the inner wall or the bottom surface that impedes fluid movement. As illustrated in Fig. 3, for example, the kneading efficiency is increased by using a cylindrical container with irregularities on the inner sidewall (a container with a cloverleaf cross section viewed from above). Fig. 3 is a Y - Y cross section view of Fig. 1. The cylindrical container 10 shown in Fig. 3 has a plurality of concave surfaces 12 on the inner periphery of its sidewall 11 and the centers of curvature 12a of the adjacent concave-curved surfaces are at different positions, whereby a convex-shaped crest 13 that protrudes toward the interior of the cylindrical container 10 is formed between the adjacent concave-curved surfaces 12. As shown in Figs. 3 and 4, the number of the convex-shaped crests 13 on the inner periphery of its sidewall may be one or more. The cylindrical container 10 may have a similar crest. Increases in the temperature of the container are monitored, and if necessary, the entire container may be cooled down and then the kneading may be resumed thereafter. This operation results in a paste such as a tooth paste in which the hemoglobin solution and the complex lipid are kneaded. Usually, when lipid powder is dispersed in an aqueous solution, it takes time to remove foam. With the method of the present invention, however, the resulting paste has hardly any foam because the foam is removed by the centrifugal force of revolution. The paste is processed with the same machine. When pure water or saline is added to the paste, sealed in and kneaded by the above-mentioned machine for about another minute under similar conditions, the paste is dispersed to become a fluid dispersion of hemoglobin vesicles, which simplifies the subsequent operations. In case lipid which is not completely dispersed by the kneading or protein which is denatured and become insoluble remain, they are removed at this step by light centrifugation (about 2000 - 5000 x g) or by filtration. To remove hemoglobin which is not encapsulated, the fluid dispersion of hemoglobin vesicles is treated with an ultrafiltration membrane (molecular weight cut-off 1000 kDa) or ultracentrifugation (about 50000 x g) to remove the supernatant followed by re-dispersion of the precipitate in saline. The resulting hemoglobin vesicle is characterized regarding recovery yield of hemoglobin, average particle size and measurement of concentrations of components. Depending on the kneading conditions, the resulting hemoglobin has a recovery yield of hemoglobin at 50 - 70%, an average particle size of 200 - 400 mm and a weight ratio of hemoglobin to complex lipid of 1.2 or more.

A kneading machine based on planetary movement with revolution and rotation is commercially available from many companies as various models, which differ in the rates of revolution and rotation and their ratio. As the dimensions of the machine vary depending on the kneading capacity (from about 10 mL to 10 L), the larger the machine is, the longer the radius is, and the greater the kneading efficiency is with the same rate of movement. Therefore, the optimum rate for kneading differs for different models. In general, machines which run in the range of a rate of revolution of 200 - 300 rpm and at a rate of rotation of 100 - 3000 rpm are commercially available, and thus kneading operations suitable for vesicle preparation may be performed under these conditions. It is preferable to set the rate of revolution below 2000 rpm to minimize the denaturation of components by heat and to complete the operation in as soon as possible. Also, the higher the concentration of the hemoglobin solution is, the higher the viscosity is (6, 11 and 29 cP at 35, 40, 45 and 45 g/dL, respectively). As for the control of particle size, it is important to have a strong shear stress. In order to control particle size, it is advantageous to use a hemoglobin solution with high viscosity. The strong shear stress, on the contrary, tends to invite a large amount of heat and denatured protein. When the concentration of the hemoglobin solution is too low, the hemoglobin content encapsulated in the resulting particle is reduced. In view of these considerations, it is preferable to set the concentration of the hemoglobin solution at 35 - 45 g/dL. It is desirable to set the viscosity of the hemoglobin solution at 5 cP or higher as measured at 23°C under a condition of shear velocity at 1000 s⁻¹. The fluid dispersion of hemoglobin vesicles is preserved after sealing in a container which does not permeate gas (glass container) as a formulation in a CO-bound form, subsequent to adjusting the concentration, or alternatively, after sealing as a formulation converted in deoxy-form with a nitrogen gas aeration or a gas exchange unit such as an artificial lung, subsequent to carrying out CO photo dissociation and removal of oxygen and adjusting the concentration. When the particle size is sufficiently small, it is possible to filtrate through a sterilized filter with a pore size of 0.22 micrometers. In case it is impossible to carry out filter sterilization, the entire process may be kept in a sterile atmosphere, or a predetermined amount of beta-propiolactone may be supplemented in the final step of process. The principle of sterility for this reagent is to denature DNA by direct binding. As the reagent may also denature protein by direct binding, it is possible to suppress denaturation of hemoglobin (conversion to methemoglobin) to some degree by supplementing beta-propiolactone in a state where oxygen dissolved inside the hemoglobin vesicles is completely excluded (in a carbonyl hemoglobin or deoxyhemoglobin state) in order to protect the hemoglobin.

As for complex lipid components of the phospholipid vesicle, four ingredients, a phosphatidylcholine-type phospholipid, cholesterol, a negatively-charged lipid and a lipid bound with polyethylene glycol, are appropriate as major components. The complex lipids are prepared with unlimited combinations and composition ratios. As for the phosphatidylcholine-type phospholipid, fatty acids, which are bound to hydroxyl groups at 1 -and 2 - positions of glycerol backbones by ether bonds, are preferably saturated in order to suppress lipid peroxidation. It is preferable to use 1,2-dimyristoyl-sn-glycero-3-phosphatidylcholine (DMPC), 1,2-dipalmitoyl-sn-glycero-3-phosphatidylcholine (DPPC), 1,2-distearoyl-sn-glycero-3-phosphatidylcholine (DSPC), 1-palmitoyl-2-myristoyl-sn-glycero-3-phosphatidylcholine (PMPC), 1-stearoyl-2-myristoyl-sn-glycero-3-phosphatidylcholine (SMPC), or hydrogenated lecithin derived from soybean (HSPC). With unsaturated type phospholipids, it is preferable to use a phospholipid bound with oleic acid, a relatively stable fatty acid, such as 1-stearoyl-2-oleoyl-sn-glycero-3-phosphatidylcholine (SOPC), 1-palmitoyl-2-oleoyl-sn-glycero-3-phosphatidylcholine (POPC), and the like. Of these phospholipids, DPPC is supposed to be preferable, considering the stability of the resulting vesicles, as well as accumulation subsequent to capture by macrophages, which should be avoided. In view of the ease of production, it is desirable to use a phosphatidylcholine-type lipid with phase transition temperature below that of DPPC (Tc = 41°C) such as SMPC (Tc = 30°C), PMPC (28°C), DMPC (23°C), SOPC (6°C) and POPC (-3°C), because of their favorable characteristics regarding dispersion and control of particle size. Regarding the negatively-charged lipid, it is preferable to use 1,5-0-dihexadecyl-N-succinyl-L-glutamate. Conventionally, with a phosphatidylcholine-type lipid with low phase transition temperature, it is regarded that the resulting vesicle structure is unstable. It is necessary, however, to judge whether the structure is sufficiently stable, from the viewpoint of actual intended use.

Different procedures can be employed to prepare the dried powder of complex lipid to be kneaded with the dense hemoglobin solution as follows: (1) a dried powder obtained by first dissolving all the components in a predetermined organic solvent, followed by rapid removal of the organic solvent by the CRUX method; (2) a dried powder obtained by first dissolving all the components in t-butanol or benzene, followed by freeze-drying; (3) a dried vesicle powder obtained by dissolving the components in an organic solvent such as t-butanol or ether, contacted with water, forming vesicles in water by removing the organic solvent and freeze-drying the vesicles; (4) a dried vesicle powder obtained by dispersing the complex lipid powder obtained by procedure (1) or (2) in water to form vesicles and freeze-drying the vesicles; and (5) a freeze-dried powder of lipid gel obtained by hydrating the complex lipid powder obtained by procedure (1) or (2) with the above-mentioned kneading machine. However, it takes a long time to freeze-dry an aqueous solution, and it also costs an enormous amount of labor and electric power to run a freeze drying apparatus. In view of efficiency, it is desirable to use the complex lipid powder obtained by procedure (1) or (2) as it is. In the embodiments of the present invention, sufficient efficiency in encapsulating hemoglobin and controlling average particle size is achieved by kneading the dried complex lipid powder obtained by procedure (1) or (2) directly with the dense hemoglobin solution.

### [Examples]

### Example 1

1,2-dipalmitoyl-sn-glycero-3-phosphatidylcholine (DPPC), cholesterol, 1,5-O-dihexadecyl-N-succinyl-glutamate (DHSG) and 1,2-distearoyl-sn-glycero-3-phosphatidylethanolamine-N-poly(oxyethylene)5000 (DSPE-PEG5000, molecular weight of PEG chain: 5000) with molar ratio of 5:4:0.9:0.03, respectively, and total weight of 10 g were dissolved as complex lipids in 1 dL of t-butanol in a 0.5 L eggplant shaped flask. The solution was frozen with a dry ice/methanol mixed refrigerant and applied to a freeze-drying machine (TOKYO RIKAKIKAI CO., LTD., FD-1000) for 24 hours to obtain a complex lipid powder. Ten grams of the powder was put in a cylindrical container (outer diameter: 76mm; height: about 93 mm, with irregularities on the inner wall and a cloverleaf cross section viewed from above) made of Teflon (registered mark), and supplemented with highly purified human hemoglobin solution (HbCO form bound with carbon monoxide, 45 g/dL, 0.4 dL, pH 7.4). The weight ratio of hemoglobin (18 g) and lipid (10 g) was 1.8:1. The container was sealed with an inner cap and applied to a kneading machine (rotation and revolution mixer, THINKY CORPORATION, ARE-310) to be kneaded with revolution at 1500 rpm and rotation at 600 rpm for 3 minutes. After cooling for 3 minutes, the gas phase in the container was completely replaced with carbon monoxide gas and the container was sealed. The operation of kneading with revolution at 1500 rpm and rotation at 600 rpm for 3 minutes and cooling for 3 minutes was repeated a total of 30 times (90 minutes) to complete kneading treatment. At that time, the temperature of the paste was as high as about 40 °C by the shear stress during the kneading and the heat transferred from the machine. Thus, it was judged as important to cause the paste to react with carbon monoxide to suppress denaturation of hemoglobin. A dense and homogeneous paste with a dark red color was obtained. No lipid lumps or foam was observed. Then, 0.50 dL of cooled saline was added and run on the kneading machine for 15 seconds to reduce the viscosity of the paste and to make a fluid dispersion. The fluid dispersion was further diluted two-fold with saline and centrifuged (3000 rpm, 30 minutes, Hitachi Koki Co., Ltd., CF12RX) to obtain a precipitate of a fraction with large particle size and a precipitate of partially denatured hemoglobin. The supernatant phase was collected into an ultracentrifuge tube (a container of 230 mL), filled with saline and spun at 50,000 rpm for 30 minutes with an ultracentrifuge (Hitachi Koki Co., Ltd., CP90WX). Measuring the hemoglobin concentrations and volumes of the fluid dispersion before spinning and the supernatant after spinning, the encapsulating efficiency was calculated to be about 65%. The particle size was measured with a nanoparticle analyzer by HORIBA, Ltd. to be about 270 nm. The percentage of methemoglobin content was calculated based on the soret band spectrum and confirmed to be less than 3%. The precipitate obtained by the ultracentrifugation was dispersed in saline again, the hemoglobin concentration was adjusted to 2.5 g/dL, and put into a 2 L eggplant shaped flask in 2 dL increments to light dissociate CO gas and to convert the precipitate to oxygen-bound hemoglobin by running with a rotary evaporator and being illuminated from outside with a halogen lamp for 45 minutes. The obtained solution was separated again with an ultracentrifuge. The precipitate was dispersed in saline, and the hemoglobin concentration was adjusted to 10 g/dL. The lipid concentration of the dispersion was 6.8 g/dL and the weight ratio of hemoglobin to lipid was 1.5. Aliquots were prepared in vials and sealed with caps made of butyl rubber. The hemoglobin was converted to deoxy-form by nitrogen gas aeration to exclude oxygen. Then, beta-propiolactone was supplemented at a concentration of 0.5% or lower to perform sterilization.

### [Comparative Example 1]

Four lipid components were dissolved in t-butanol to be a homogeneous solution, and freeze-dried to obtain 10 g of complex lipid powder as described in connection with Example 1. The powder was added to 0.4 dL of 45 g/dL hemoglobin solution and mixed for 24 hours with a stirrer and a stirrer tip (length: 2 cm). A large amount of lipid lumps which was not dispersed was found and the solution was too dense to control the particle size using the conventional Extrusion Method.

### [Comparative Example 2]

As described in connection with Example 1, four lipid components were dissolved in t-butanol to make a homogeneous solution, and freeze-dried to obtain complex lipid powder. Five grams of the powder was supplemented to 1 dL of 45 g/dL hemoglobin solution, and mixed for 12 hours with a stirrer and a stirrer tip (length: 2 cm). Some lipid lumps were found. The mixture was treated with the Extrusion method using an extruder of Lipex Biomembranes, Inc. and the particle size was controlled by step wise filtration with filters having pore sizes 3.0, 1.0, 0.8, 0.65, 0.45, and 0.22 micrometers in this order. At this time, the viscosity at 25 °C was measured using a rheometer (MCR-300, Anton Paar GmbH) to be 62 cP (shear velocity at 1000 s⁻¹), 70 cP (shear velocity at 100 s⁻¹) and 98 cP (shear velocity at 10 s⁻¹). The amount recovered was 0.8 dL and 0.2 dL was lost during the processes. The encapsulation efficiency was as low as 20%. Adjusting hemoglobin concentration at 10 g/dL, 70 mL of hemoglobin vesicle dispersion was obtained.

### [Comparative Example 3]

In the procedures described in connection with Example 1, oxyhemoglobin, hemoglobin bound with oxygen, was used to prepare a complex lipid powder which was kneaded by similar procedures. When the paste was diluted and centrifuged (200 rpm, 60 minutes), a large amount of insoluble precipitate with medium brown color was found. The percentage of methemoglobin in supernatant hemoglobin increased to about 10%. Thus, it was judged that stabilization of hemoglobin by converting into carbon monoxide-bound hemoglobin or deoxyhemoglobin is an indispensable step.

### [Example 2]

Using the same species of lipids as Example 1, DPPC, cholesterol, DHSG and DSPE-PEG5000 at a molar ratio of 5:4:0.9:0.03 and a total weight of 10 g were dissolved in t-butanol in an eggplant shaped flask. Freezing with liquid nitrogen and freeze-drying were carried out to obtain a complex lipid powder. Then, the complex lipid was put into the same cylindrical container made of Teflon (registered mark) as that used in Example 1, and supplemented with 0.5 dL of ultrapure water. As the weight of the negatively-charged lipid DHSG was 1.13 g, corresponding to 1.48 millimol, 0.074 mL of 1N NaOH solution was added to neutralize the negatively charged lipid DHSG with the same amount of NaOH. Then, the container was sealed with the inner cap and applied to a kneading machine (rotation and revolution mixer, THINKY CORPORATION, ARE-310) to perform kneading with revolution at 2000 rpm and rotation at 800 rpm for 5 minutes. After cooling for 5 minutes, kneading was carried out again for 5 minutes so that kneading was carried out for 10minutes in total. An obtained lipid lamellar gel was frozen with liquid nitrogen and freeze dried to obtain freeze-dried powder of the lipid gel. 0.4 dL of HbCO solution was added to 10 g of the complex lipid similar to the procedures described in connection with Example 1, and the container was sealed with CO and kneading was carried out using the kneading machine (rotation and revolution mixer, revolution at 1500 rpm and rotation at 600 rpm) to obtain a kneaded paste of hemoglobin solution and freeze dried lipid powder. Saline was added to the paste and diluted, the paste was dispersed in the same manner, and aggregates and the like were separated and removed by centrifugation. The upper layer solution was further separated by ultracentrifugation and the concentration and volume of hemoglobin which was not encapsulated was measured, to calculate the encapsulation efficiency as about 60%. The precipitated hemoglobin vesicles were dispersed in saline again and hemoglobin concentration was adjusted to 10 g/dL, and aliquots of 0.50 dL were dispensed in 1 dL vials and sealed. Aeration was carried out with carbon monoxide gas to exclude dissolved oxygen, and the hemoglobin was completely converted to that which is bound to carbon monoxide. Then, beta-propiolactone was added at a concentration of 0.5% or lower to perform sterilization.

### [Example 3]

Complex lipid powder was prepared by Nippon Fine Chemical Co., Ltd. with DPPC, cholesterol, DHSG and DSPE-PEG 5000 dissolved in an organic solvent at a molar ratio of 5:4:0.9:0.03, and the organic solvent being rapidly evaporated by the CRUX method. 10 g of the complex lipid powder was added to 0.4 dL of a dense hemoglobin solution in a manner similar to that of Example 1, and kneading with a kneading machine (rotation and revolution mixer, THINKY CORPORATION, ARE-310, revolution at 1000 rpm and rotation at 400 rpm) for 6 minutes and cooling for 3 minutes were repeated 30 times. The temperature of the paste was as low as about 30 °C. Kneading was carried out for 180 minutes in total. At that time, the viscosity of the obtained paste at 25 °C was measured using a rheometer (MCR-300, Anton Paar GmbH) to be about 2200 cP (shear velocity at 1000 s⁻¹ at 23 °C), about 10000 cP (shear velocity at 100 s⁻¹ at 23 °C) and about 52000 cP (shear velocity at 10 s⁻¹ at 23 °C). Then, the hemoglobin vesicles were collected in a manner similar to that of Example 1, and it was found that the encapsulation efficiency was about 74% and the particle size was about 280 nm. It was concluded that this was a kneading condition suitable for suppressing temperature rise of the paste and reducing protein denaturation, although kneading time became longer by slowing down the rates of revolution and rotation.

### [Comparative Example 4]

A complex lipid similar to that of Example 1 but without DHSG was prepared. DPPC, cholesterol and DSPE-PEG5000 with a molar ratio of 5:4:0.03 and a total weight of 10 g were dissolved in t-butanol in a 0.5L eggplant shaped flask. The complex lipid powder was obtained by freezing with a dry ice/methanol mixed refrigerant and applying to a freeze-drying machine (TOKYO RIKAKIKAI CO., LTD., FD-1000) for 24 hours. Ten grams of the powder was put in a cylindrical container made of Teflon (registered mark), and supplemented with highly purified human hemoglobin solution (HbCO form bound with carbon monoxide, 45 g/dL, 0.4 dL, pH 7.4). Kneading was carried out in a manner similar to that of Example 3. The encapsulation efficiency was only 26% and the weight ratio of hemoglobin to lipid was a remarkably low value of 0.94. Accordingly, DHSG was found to be indispensable to increase recovery yield of hemoglobin in the experiments to encapsulate hemoglobin described in the present application. Although the effect of DHSG on recovery yield of hemoglobin is clarified as in the above description, there is a possibility that the effect of DHSG on the recovery yield might be affected by the species of the functional substance to be encapsulated and kneading conditions. The present invention, therefore, does not intend to exclude the use of complex lipid powder which does not comprise DHSG.

### [Example 4]

Pyridoxal 5'-phosphate of 2.5-fold moles with respect to carbonyl hemoglobin was added to a solution of carbonyl hemoglobin. Ten grams of complex lipid obtained by the CRUX method as described in Example 3 was added to 0.4 dL (45 g/dL, pH7.4) of the hemoglobin solution, and kneading with a kneading machine (rotation and revolution mixer, THINKY CORPORATION, ARE-310, revolution at 1000 rpm and rotation at 400 rpm) for 6 minutes and cooling for 3 minutes were repeated 30 times in a manner similar to that of Example 3. Kneading was carried out for 180 minutes in total. The encapsulation efficiency was about 65% and the particle size was about 250 nm. Removal of carbon monoxide and oxygen was performed as described in connection with Example 1, and aliquots of 0.50 dL were dispensed in 1 dL vials. Then, beta-propiolactone was added at a concentration of 0.5% or lower to complete sterilization.

### [Example 5]

In a manner similar to that of Example 1, a complex lipid powder was prepared by Nippon Fine Chemical Co., Ltd. with DPPC, cholesterol, DHSG and DSPE-PEG 5000 dissolved in an organic solvent at molar ratio of 5:4:0.9:0.03, and the organic solvent being rapidly evaporated by the CRUX method. In this example, the concentration of hemoglobin bound to carbon monoxide was reduced to 40 g/dL. Ten grams of the complex lipid powder was added to 0.4 dL of a hemoglobin solution, and kneading with a kneading machine (rotation and revolution mixer, THINKY CORPORATION, ARE-500, revolution at 800 rpm and rotation at 784 rpm) for 30 minutes was repeated 3 times. Kneading was carried out for 90 minutes in total. Then, the paste was diluted with saline in a manner similar to that of Example 1 and the precipitates were removed by centrifugation (3000 rpm, 30 min.). The supernatant was separated by ultracentrifugation to collect hemoglobin vesicles, which were dispersed again to obtain a hemoglobin vesicle dispersion. The encapsulation efficiency was about 80% and the particle size was about 280 nm.

### [Comparative Example 5]

The amounts of lipids and hemoglobin were increased two-fold compared to those of Example 5. Twenty grams of complex lipid powder and 0.8 dL of hemoglobin solution (40 g/dL) were put in the container described in connection with Example 1, and kneading was carried out for 90 minutes in total in the manner described in connection with Example 5. Then, the paste was diluted with saline in a manner similar to that of Example 1 and the precipitates were removed by centrifugation (3000 rpm, 30 min.). The supernatant was separated by ultracentrifugation to collect hemoglobin vesicles, which were dispersed again to obtain a hemoglobin vesicle dispersion. As the encapsulation efficiency was reduced to about 50%, it was shown that the yield varied depending on the amount of materials placed in the container.

### [Example 6]

In this example, a condition was examined that used the same amount of lipids and hemoglobin as Comparative Example 5, but in a larger container. 20 g of the complex lipid powder and 0.8 dL of hemoglobin solution (40 g/dL) were put in a container larger than the one described in Example 1 (outer diameter: 90 mm; height: about 108 mm), kneading with a kneading machine (rotation and revolution mixer, THINKY CORPORATION, ARE-500, revolution at 800 rpm and rotation at 784 rpm) for 30 minutes was repeated 3 times or 90 minutes in total Then, the paste was diluted with saline in a manner similar to that of Example 1 and the precipitates were removed by centrifugation (3000 rpm, 30 min.). The supernatant was separated by treatment with ultrafiltration membrane (molecular weight cut-off 1000kDa; membrane area: 0.1 m²; Biomax V screen, EMD Millipore Corporation) to remove hemoglobin which was not encapsulated and to obtain a hemoglobin vesicle dispersion. The encapsulation efficiency was about 75%. As the strongest shear stress was generated on the contact area of the paste and the inner wall of the rotating container, the large area of contact was considered to be effective in improving encapsulation efficiency.

### [Example 7]

In this example, PMPC, which has a lower phase transition temperature than DPPC, was used as a phosphatidylcholine-type phospholipid. PMPC, cholesterol, DHSG and SDPE-PEG5000 with a molar ratio of 5:4:0.9:0.03 were dissolved in t-butanol, and freeze-dried to obtain 4 g of dried lipid powder. The powder was supplemented with dense hemoglobin solution (40 g/dL, 0.2 dL), and production of hemoglobin vesicles was attempted in the same manner as in Example 1. After kneading for 3 minutes with the kneading machine (rotation and revolution mixer, THINKY CORPORATION, ARE-310, revolution: 1500 rpm; rotation: 600 rpm) was repeated 30 times, the solution was diluted 4 fold with saline and centrifuged (2000 rpm, 60 minutes). The amount of precipitate in large particle size fraction which was not completely dispersed was significantly reduced compared with Example 1. The particle size was 200 -300 nm, which means that the particle size was controlled to be smaller. Thus, it was judged that production is facilitated when a phosphatidylcholine-type phospholipid with a lower phase transition temperature is used.

### [Example 8]

Stability was evaluated for the hemoglobin vesicles prepared in Example 1 with the complex lipid comprising DPPC as the major component and the hemoglobin vesicles prepared in Example 7 with the complex lipid comprising PMPC as the major component. Washed rat red blood cells were used as a comparison. For each evaluated sample, the hemoglobin concentration was adjusted to 3 g/dL and the percentage of hemolysis was measured after following treatments: (1) freezing with liquid nitrogen and subsequent thawing; (2) 5-fold dilution with distilled water; (3) hydrolysis of phospholipid by phospholipase A2; and (4) fluid movement under shear stress with a shear velocity of 1000 s⁻¹ for 2 hours. The hemoglobin vesicles or red blood cells were precipitated by ultracentrifugation and the percentage of hemolysis was calculated from hemoglobin concentration and the volume of the supernatant. N = 3, or triplicate samples were prepared for each treatment, and average values ± standard deviation were calculated. After treatment (1), 75.9±9.2% of the red blood cells were hemolytic, while 33.7±4.7% of the DPPC-based hemoglobin vesicles and 33.3±4.2% of the PMPC-based hemoglobin vesicles were hemolytic. After treatment (2), the red blood cells were highly hemolytic at 89.0±6.6% while the DPPC- and PMPC-based hemoglobin vesicles were less hemolytic at 0.9±0.4% and 0.6±0.4%, respectively. After treatment (3), 6.9±1.3% of the red blood cells were hemolytic, while 0.9±0.7% of the DPPC-based hemoglobin vesicles and 0.5±0.1% of the PMPC-based hemoglobin vesicles were hemolytic. After treatment (4), 4.8±0.3% of the red blood cells were hemolytic, while less than 1% of the DPPC- and PMPC-based hemoglobin vesicles were hemolytic. Thus, it was shown that both of the DPPC- and PMPC-based hemoglobin vesicles are structurally extremely stable. Although DPPC and PMPC have different phase transition temperatures, it was also shown that there are no notable differences in the stabilities of DPPC- and PMPC-based hemoglobin vesicles.

### [Example 9 (outside the scope of the claims)]

As a model of low molecular weight functional substances, a fluorescent compound 5 (6)-carboxyfluorescein (CF) solution was prepared by dissolving CF at 10 mM in a phosphate buffered solution (pH 7.4). A complex lipid powder was prepared by Nippon Fine Chemical Co., Ltd. with DPPC, cholesterol, DHSG and DSPE-PEG 5000 dissolved in an organic solvent at molar ratio of 5:4:0.9:0.03, and the organic solvent being rapidly evaporated by the CRUX method. Ten grams of the complex lipid powder was added to 40 mL of the CF solution, put in the cylindrical container described in connection with Example 1 and kneading with a kneading machine (rotation and revolution mixer, THINKY CORPORATION, ARE-310, revolution at 1000 rpm and rotation at 400 rpm) for 6 minutes and cooling for 3 minutes were repeated 30 times. After the repeated kneading and cooling, a paste like a hand cream was obtained. Viscosity of the paste at 25 °C was measured using a rheometer (MCR-300, Anton Paar GmbH) to be about 1020 cP (shear velocity at 1000 s⁻¹ at 23 °C), 4600 cP (shear velocity at 100 s⁻¹ at 23 °C) and about 36800 cP (shear velocity at 10 s⁻¹ at 23 °C). About 1 g of the resulting paste was supplemented with about 10 mL of saline and mixed by shaking to obtain a vesicle dispersion. The vesicles were precipitated by ultracentrifugation (100000 x g, 1 hour). The resulting supernatant CF concentration and volume, as well as CF concentration and the entire volume before the ultracentrifugation, were used to calculate encapsulation efficiency of CF as 85%. The average particle size of the vesicle was 800 nm. It was considered that the reason why the particle size was not sufficiently small was because the viscosity of the CF solution was lower than that of the hemoglobin solutions and the shear stress was not strong enough. It was concluded to further extend kneading time in order to control the particle size to be smaller. A disclosed in the Japanese translation of PCT international application No.: 2008-542360, vesicles may generally encapsulate pharmaceutical reagents to be applied for use in the treatment, prevention, diagnosis and the like of disease conditions or therapeutics. A pharmaceutical reagent may generally be selected from a group of anti-virus agents, anti-microbe agents, anti-bacterium agents, anti-fungus agents, anti-neoplasm agents, antiinflammatory agents, radiolabelling agents, radiopaque compounds, fluorescent compounds, pigment compounds, polynucleotides, anticancer agents, cell growth factors, hematopoietic factors (erythropoietin, G-CSF), physiologically active substances, and the like. The lipids which encapsulate these substances defined in the claims are those described above, A special treatment may be employed such as absorbing or fixing by chemical bonding to a water-soluble polymer (such as albumin, gelatin, etc.), followed by stably encapsulating the aqueous solution dissolving a complex of the water-soluble polymer-functional substance in vesicles. It is possible to stably encapsulate hemoglobin in the vesicles, by using an auxiliary substance such as a water-soluble polymer to disperse the functional substance in the water.

### [Example 10 (outside the scope of the claims)]

Albumin physically absorbs various lipophilic functional substances, and thus has a potential to be a carrier for the various lipophilic functional substances. In this example, therefore, vesicles encapsulating a dense albumin solution were prepared. Ten grams of the lipid mixture of DPPC, cholesterol, DHSG and PEG-DSPE described in connection with Example 1 in a molar ratio of 5:4:0.9:0.03 was put in the cylindrical container described in connection with Example 1. Then, 40 mL of human serum albumin (Baxter International Inc., 25 g/dL) was added and kneading with a kneading machine (rotation and revolution mixer, THINKY CORPORATION, ARE-310, revolution at 1000 rpm and rotation at 400 rpm) for 6 minutes and cooling for 3 minutes were repeated 30 times. Cooled saline, 120 mL, was added to the obtained paste and kneading was carried out for a minute to reduce viscosity. Vesicles were precipitated by ultracentrifugation (50,000 x g, one hour) and albumin which was not encapsulated was removed. The precipitated vesicles were dispersed again to obtain vesicles encapsulating albumin.

### [Example 11 (outside the scope od the claims)]

In this example, vesicles carrying curcumin as a lipophilic functional substance were prepared. DMPC, cholesterol, DHSG,PEG-SDPE and curcumin in a molar ratio of 5:1:1:0.03:1 were dissolved in heated t-butyl alcohol and freeze dried to obtain a powder mixed with curcumin and lipids. 20 g of the mixed powder was put in the cylindrical container described in connection with Example 1, supplemented with 0.8 dL of phosphate buffered saline (pH 7.4) and kneaded with a kneading machine (rotation and revolution mixer, THINKY CORPORATION, ARE-500, revolution at 800 rpm and rotation at 784 rpm) for 150 minutes in a nitrogen atmosphere to obtain a vesicle dispersion carrying curcumin.

The embodiments above are some of the many possible embodiments which illustrate the application of the principle of the present invention.

### [List of Reference Numerals]

- 10: cylindrical container
- 11: inner periphery of its sidewall
- 12: concave-curved surface
- 13: crest
- 20: center axis (axis of rotation)

## Claims

1. A method for producing vesicles encapsulating a functional substance, comprising the steps of:
(a) putting the functional substance, lipid and water in a cylindrical container; and
(b) producing lipid vesicles which comprise the lipid as a main component and which encapsulate the functional substance therein, by kneading the contents of the cylindrical container with simultaneous rotational movement of the cylindrical container around its center axis together with revolutionary movement of the cylindrical container about a predetermined axis of revolution;
wherein the axis of rotation is not on parallel with the axis of revolution;
wherein the functional substance is hemoglobin; and
wherein the lipid is comprised of a phosphatidylcholine-type phospholipid, cholesterol, a negatively-charged lipid, and a lipid bound with polyethylene glycol.

2. The method according to Claim 1, wherein the contents of the cylindrical container in step (a) are prepared by adding the lipid in an aqueous solution comprising the functional substance.

3. The method according to Claim 1, wherein the contents of the cylindrical container in step (a) are prepared by dispersing a lipid powder in the water, the lipid powder being obtained by drying the lipid comprising the functional substance.

4. The method according to Claim 1, 2 or 3, further comprising the steps of:
(c), after step (b), adding water or saline to a liquid or paste in the cylindrical container; and
(d), after step (c), reducing the viscosity of the liquid or paste in the cylindrical container by further rotating the cylindrical container around the center axis while revolving the cylindrical container about the predetermined axis of revolution.

5. The method according to Claim 4, further comprising a step
(e), after step (d), removing the functional substance which is not encapsulated with the lipid by applying an ultrafiltration membrane technique or ultracentrifugation technique to the fluid or paste in the cylindrical container.

6. The method according to Claim 1, 2, 3, 4 or 5, wherein, in step (b), the step of kneading the aqueous solution by rotating the cylindrical container around the center axis while the cylindrical container revolves about the predetermined revolution axis is implemented multiple times, and during the interval between two consecutive kneading steps, a cooling treatment is performed to cool down the fluid or paste by stopping at least one of rotation and revolution of the cylindrical container or by reducing the rate of at least one of rotation and revolution of the cylindrical container.

7. The method according to Claim 1, 2, 3, 4, 5 or 6, wherein the cylindrical container has multiple concave-curved surfaces on the inner periphery of its sidewall and the centers of curvature of adjacent concave-curved surfaces are at different positions, whereby a convex-shaped crest that protrudes towards the interior of the cylindrical container is formed between the adjacent concave-curved surfaces.

8. The method according to Claim 1, wherein the aqueous solution of step (a) is prepared by adding 15 g or more of a complex lipid powder as the lipid per 1 dL of an aqueous solution of hemoglobin in which 30 - 50 g/dL of hemoglobin is dissolved; and
wherein the hemoglobin is carbonyl hemoglobin with its heme at a divalent iron state or deoxyhemoglobin with its heme at a divalent iron state.

9. The method according to Claim 1, wherein the functional substance is hemoglobin and the aqueous solution of step (a) is prepared by adding 15 g or more of a complex lipid powder as the lipid per 1 dL of an aqueous solution of hemoglobin in which 30 - 50 g/dL of hemoglobin is dissolved; and
further comprising a step of removing contaminating unstable proteins by degeneration with a treatment that heats the aqueous solution of hemoglobin to 50°C or higher for five hours or longer before adding the lipid to the aqueous solution of hemoglobin, and removing the contaminating unstable proteins with an ultrafiltration membrane or centrifugation,
wherein the removing step is implemented in order to reduce the occurrence of insoluble matter of the degenerated protein in the course of the kneading treatment in step (b).

10. The method according to Claim 1, 2, 3, 4, 5, 6, 7, 8 or 9, wherein the lipid is comprised of a phosphatidylcholine-type phospholipid of 1,2-dipalmitoyl-sn-glycero-3-phosphatidylcholine, cholesterol, a negatively-charged lipid of 1, 5-O-dihexadecyl-N-succinyl-glutamate, and a lipid bound with polyethylene glycol of 1, 2-distearoyl-sn-glycero-3-phosphatidylethanolamine-N-Poly(oxyethylene) 5000 (molecular weight of the polyethylene glycol chain: 5,000).

11. The method according to Claim 1, 2, 3, 4, 5, 6, 7, 8 or 9, wherein the gel-liquid phase transition temperature of the phosphatidylcholine-type phospholipid is 30°C or lower.

12. The method according to Claim 1, 2, 3, 4, 5, 6, 7, 8, 9, 10 or 11, comprising the steps of producing dried lipid powder from a lipid lamellar gel as the lipid, wherein the step of producing the dried lipid powder comprises steps of:
producing an aqueous solution of the lipid by adding 15 g/dL or more of the lipid powder in pure water which is comprised of substantially no solute;
kneading the aqueous solution of lipid within a cylindrical container, rotating the cylindrical container around its center axis together with revolving the cylindrical container about a predetermined axis of revolution; and
obtaining the dried lipid powder from the lipid lamellar gel by freeze-drying the kneaded aqueous solution of lipid.

13. The method according to Claim 5, comprising a step of
after (e), adding beta-propiolactone to the fluid or paste that is obtained after removing the functional substance which is not encapsulated with the lipid.

14. A vesicle produced by the method according to any one of claims 1 to 13, wherein said vesicle comprises of a phosphatidylcholine-type phospholipid, a negatively-charged lipid, cholesterol, and a lipid bound with polyethylene glycol, wherein the gel-liquid phase transition temperature of the phosphatidylcholine-type phospholipid is below that of 1 ,2-dipalmitoyl-sn-glycero-3-phosphatidylcholine.

## Patentansprüche

1. Verfahren zur Herstellung von Vesikeln, die eine funktionelle Substanz einkapseln, folgende Schritte umfassend:
(a) Einfüllen der funktionellen Substanz, des Lipids und des Wassers in einen zylindrischen Behälter; und
(b) Herstellen von Lipidvesikeln, die das Lipid als Hauptkomponente enthalten und die darin enthaltene funktionelle Substanz einkapseln, indem der Inhalt des zylindrischen Behälters bei gleichzeitiger Rotationsbewegung des zylindrischen Behälters um seine Mittelachse zusammen mit einer Umwälzbewegung des zylindrischen Behälters um eine vorgegebene Umwälzungsachse geknetet wird;
wobei die Drehachse nicht parallel zur Umwälzungsachse ist;
wobei die funktionelle Substanz Hämoglobin ist; und
wobei das Lipid aus einem Phospholipid vom Phosphatidylcholin-Typ, Cholesterin, einem negativ geladenen Lipid und einem mit Polyethylenglycol gebundenen Lipid besteht.

2. Verfahren nach Anspruch 1, wobei der Inhalt des zylindrischen Behälters in Schritt (a) durch Zugabe des Lipids in einer wässrigen Lösung hergestellt wird, welche die funktionelle Substanz umfasst.

3. Verfahren nach Anspruch 1, wobei der Inhalt des zylindrischen Behälters in Schritt (a) durch Dispergieren eines Lipidpulvers in Wasser hergestellt wird, wobei das Lipidpulver durch Trocknen des Lipids erhalten wird, das die funktionelle Substanz umfasst.

4. Verfahren nach Anspruch 1, 2 oder 3, ferner folgende Schritte umfassend:
(c) nach Schritt (b) Hinzufügen von Wasser oder Salzlösung zu einer Flüssigkeit oder Paste in dem zylindrischen Behälter; und
(d) nach Schritt (c) Verringern der Viskosität der Flüssigkeit oder Paste in dem zylindrischen Behälter durch weiteres Drehen des zylindrischen Behälters um die Mittelachse, während der zylindrische Behälter um die vorbestimmte Umwälzungsachse gedreht wird.

5. Verfahren nach Anspruch 4, ferner umfassend einen Schritt (e) nach Schritt (d), Entfernen der funktionellen Substanz, die nicht mit dem Lipid eingekapselt ist, durch Anwenden einer Ultrafiltrationsmembrantechnik oder Ultrazentrifugationstechnik auf das Fluid oder die Paste in dem zylindrischen Behälter.

6. Verfahren nach Anspruch 1, 2, 3, 4 oder 5, wobei in Schritt (b) der Schritt des Knetens der wässrigen Lösung durch Drehen des zylindrischen Behälters um die Mittelachse während sich der zylindrische Behälter um die vorbestimmte Umwälzungsachse dreht, mehrmals ausgeführt wird, und während des Intervalls zwischen zwei aufeinanderfolgenden Knetschritten eine Kühlbehandlung durchgeführt wird, um das Fluid oder die Paste abzukühlen, indem mindestens eines aus Drehung und Umwälzung des zylindrischen Behälters gestoppt wird oder mindestens eines aus Drehgeschwindigkeit und Umwälzung des zylindrischen Behälters verringert wird.

7. Verfahren nach Anspruch 1, 2, 3, 4, 5 oder 6, wobei der zylindrische Behälter mehrere konkav gekrümmte Oberflächen am Innenumfang seiner Seitenwand aufweist und sich die Krümmungsmittelpunkte von benachbarten konkav gekrümmten Oberflächen an verschiedenen Positionen befinden, wobei zwischen den benachbarten konkav gekrümmten Flächen ein konvex geformter Scheitel entsteht, der zum Inneren des zylindrischen Behälters vorsteht.

8. Verfahren nach Anspruch 1, wobei die wässrige Lösung von Schritt (a) durch Zugabe von 15 g oder mehr eines komplexen Lipidpulvers als Lipid pro 1 dl einer wässrigen Lösung des Hämoglobins, in denen 30 - 50 g/dl Hämoglobin aufgelöst wird, hergestellt wird; und wobei das Hämoglobin Carbonyl-Hämoglobin ist, wobei sich sein Häm in einem zweiwertigen Eisenzustand befindet, oder Desoxyhämoglobin, wobei sich sein Häm in einem zweiwertigen Eisenzustand befindet.

9. Verfahren nach Anspruch 1, wobei die funktionelle Substanz Hämoglobin ist und die wässrige Lösung von Schritt (a) durch Zugabe von 15 g oder mehr eines komplexen Lipidpulvers hergestellt wird, als Lipid pro 1 dl einer wässrigen Lösung von Hämoglobin, in der 30 - 50 g/dl Hämoglobin gelöst werden; und
ferner umfassend einen Schritt des Entfernens kontaminierender instabiler Proteine durch Degeneration mit einer Behandlung, die die wässrige Lösung von Hämoglobin fünf Stunden oder länger auf 50 °C oder höher erhitzt, bevor das Lipid der wässrigen Lösung von Hämoglobin zugesetzt wird, und Entfernen der kontaminierenden instabilen Proteine durch eine Ultrafiltrationsmembran oder Zentrifugation,
wobei der Entfernungsschritt durchgeführt wird, um das Auftreten von unlöslichem Material des degenerierten Proteins im Verlauf der Knetbehandlung in Schritt (b) zu verringern.

10. Verfahren nach Anspruch 1, 2, 3, 4, 5, 6, 7, 8 oder 9, worin das Lipid aus einem Phosphatidylcholin eines Phospholipid-Typs aus 1,2-Dipalmitoyl-sn-glycero-3-Phosphatidylcholin, Cholesterin, einem negativ geladenen Lipid von 1,5-O-Dihexadecyl-N-succinyl-glutamat und einem mit Polyethylenglykol gebundenen Lipid von 1,2-Distearoyl-sn-glycero-3-phosphatidylethanolamin-N-poly (oxyethylen) 5000 (Molekulargewicht der Polyethylenglykolkette: 5.000) zusammengesetzt ist.

11. Verfahren nach Anspruch 1, 2, 3, 4, 5, 6, 7, 8 oder 9, wobei die Gel-Flüssig-Phasenübergangstemperatur des Phospholipids vom Phosphatidylcholin-Typ 30 °C oder niedriger ist.

12. Verfahren nach Anspruch 1, 2, 3, 4, 5, 6, 7, 8, 9, 10 oder 11, umfassend die Schritte des Herstellens von getrocknetem Lipidpulver aus einem lamellaren Lipidgel als Lipid, wobei der Schritt des Herstellens des getrocknetes Lipidpulvers die folgenden Schritte umfasst:
Herstellen einer wässrigen Lösung des Lipids durch Zugabe von 15 g/dl oder mehr des Lipidpulvers in reinem Wasser, das im Wesentlichen keinen gelösten Stoff enthält; Kneten der wässrigen Lipidlösung in einem zylindrischen Behälter, Drehen des zylindrischen Behälters um seine Mittelachse zusammen mit Umwälzen des zylindrischen Behälters um eine vorbestimmte Umwälzachse; und
Erhalten des getrockneten Lipidpulvers aus dem lamellaren Lipidgel durch Gefriertrocknen der gekneteten wässrigen Lipidlösung.

13. Verfahren nach Anspruch 5, umfassend einen Schritt des Hinzufügens von Beta-Propiolacton nach (e) zu der Flüssigkeit oder Paste, die nach dem Entfernen der funktionellen Substanz erhalten wird, die nicht mit dem Lipid eingekapselt ist.

14. Vesikel, hergestellt durch das Verfahren nach einem der Ansprüche 1 bis 13, wobei das Vesikel ein Phospholipid vom Phosphatidylcholin-Typ, ein negativ geladenes Lipid, Cholesterin und ein mit Polyethylenglycol gebundenes Lipid umfasst, wobei die Gel-Flüssig-Phasenübergangstemperatur des Phospholipids vom Phosphatidylcholintyp unter der von 1,2-Dipalmitoyl-sn-glycero-3-phosphatidylcholin liegt.

## Revendications

1. Procédé de production de vésicules encapsulant une substance fonctionnelle, comprenant les étapes suivantes :
(a) mise de la substance fonctionnelle, du lipide et de l'eau dans un récipient cylindrique ; et
(b) production de vésicules lipidiques qui comprennent le lipide comme composant principal et qui y encapsulent la substance fonctionnelle, par le malaxage du contenu du récipient cylindrique par un mouvement de rotation simultané du récipient cylindrique autour de son axe central conjointement avec un mouvement de révolution du récipient cylindrique autour d'un axe prédéfini de révolution ;
dans lequel l'axe de rotation n'est pas parallèle à l'axe de révolution ;
dans lequel la substance fonctionnelle est l'hémoglobine ; et
dans lequel le lipide est composé d'un phospholipide de type phosphatidylcholine, du cholestérol, d'un lipide chargé négativement et d'un lipide lié au polyéthylène glycol.

2. Procédé selon la revendication 1, dans lequel le contenu du récipient cylindrique à l'étape (a) est préparé par l'ajout du lipide dans une solution aqueuse comprenant la substance fonctionnelle.

3. Procédé selon la revendication 1, dans lequel le contenu du récipient cylindrique à l'étape (a) est préparé par dispersion d'une poudre lipidique dans l'eau, la poudre lipidique étant obtenue par séchage du lipide comprenant la substance fonctionnelle.

4. Procédé selon la revendication 1, 2 ou 3, comprenant en outre les étapes suivantes :
(c) après l'étape (b), ajout d'eau ou d'une solution saline à un liquide ou à une pâte dans le récipient cylindrique ; et
(d) après l'étape (c), réduction de la viscosité du liquide ou de la pâte dans le récipient cylindrique par la rotation supplémentaire du récipient cylindrique autour de l'axe central alors que le récipient cylindrique tourne autour de l'axe prédéfini de révolution.

5. Procédé selon la revendication 4, comprenant en outre une étape (e), après l'étape (d), l'élimination de la substance fonctionnelle qui n'est pas encapsulée par le lipide par l'application d'une technique de membrane d'ultrafiltration ou d'une technique d'ultracentrifugation au fluide ou à la pâte dans le récipient cylindrique.

6. Procédé selon la revendication 1, 2, 3, 4 ou 5, dans lequel, à l'étape (b), l'étape de malaxage de la solution aqueuse par la rotation du récipient cylindrique autour de l'axe central tandis que le récipient cylindrique tourne autour de l'axe prédéfini de révolution est réalisée plusieurs fois, tandis que pendant l'intervalle entre deux étapes consécutives de malaxage, un traitement de refroidissement est effectué pour refroidir le fluide ou la pâte par l'arrêt de la rotation et/ou de la révolution du récipient cylindrique ou par la réduction de la vitesse de rotation et/ou de révolution du récipient cylindrique.

7. Procédé selon la revendication 1, 2, 3, 4, 5 ou 6, dans lequel le récipient cylindrique comporte de multiples surfaces incurvées concaves sur la périphérie intérieure de sa paroi latérale et dans lequel les centres de courbure des surfaces incurvées concaves adjacentes sont à différentes positions, ce qui permet la formation d'une crête de forme convexe qui fait saillie vers l'intérieur du récipient cylindrique entre les surfaces incurvées concaves adjacentes.

8. Procédé selon la revendication 1, dans lequel la solution aqueuse de l'étape (a) est préparée par l'ajout d'au moins 15 g d'une poudre lipidique complexe comme lipide pour 1 dL d'une solution aqueuse d'hémoglobine dans laquelle sont dissous 30 à 50 g/dL d'hémoglobine ; et dans lequel l'hémoglobine est la carbonylhémoglobine dont l'hème est dans un état à fer divalent ou la désoxyhémoglobine dont l'hème est dans un état à fer divalent.

9. Procédé selon la revendication 1, dans lequel la substance fonctionnelle est l'hémoglobine et dans lequel la solution aqueuse de l'étape (a) est préparée par l'ajout d'au moins 15 g d'une poudre lipidique complexe comme lipide pour 1 dL d'une solution aqueuse d'hémoglobine dans laquelle sont dissous 30 à 50 g/dL d'hémoglobine ; et comprenant en outre une étape d'élimination des protéines instables contaminantes par dégénérescence à l'aide d'un traitement qui chauffe la solution aqueuse d'hémoglobine à au moins 50 °C pendant au moins cinq heures avant l'ajout du lipide à la solution aqueuse d'hémoglobine, et d'élimination des protéines instables contaminantes à l'aide d'une membrane d'ultrafiltration ou de centrifugation,
dans lequel l'étape d'élimination est réalisée afin de réduire l'apparition de matière insoluble de la protéine dégénérée au cours du traitement de malaxage à l'étape (b).

10. Procédé selon la revendication 1, 2, 3, 4, 5, 6, 7, 8 ou 9, dans lequel le lipide est constitué d'un phospholipide de type phosphatidylcholine de 1,2-dipalmitoyl-sn-glycero-3-phosphatidylcholine, du cholestérol, d'un lipide chargé négativement de 1,5-O-dihexadécyl-N-succinyl-glutamate et d'un lipide lié au polyéthylèneglycol de 1,2-distéaroyl-sn-glycéro-3-phosphatidyléthanolamine-N-poly(oxyéthylène) 5 000 (masse moléculaire de la chaîne polyéthylène glycol : 5 000).

11. Procédé selon la revendication 1, 2, 3, 4, 5, 6, 7, 8 ou 9, dans lequel la température de transition de phase gel-liquide du phospholipide de type phosphatidylcholine est inférieure ou égale à 30 °C.

12. Procédé selon la revendication 1, 2, 3, 4, 5, 6, 7, 8, 9, 10 ou 11, comprenant l'étape de production de poudre lipidique séchée à partir d'un gel lamellaire lipidique comme lipide, l'étape de production de la poudre lipidique séchée comprenant les étapes suivantes :
production d'une solution aqueuse du lipide par l'ajout d'au moins 15 g/dL de la poudre lipidique dans de l'eau pure qui ne contient pratiquement pas de soluté ;
malaxage de la solution aqueuse de lipide à l'intérieur d'un récipient cylindrique, rotation du récipient cylindrique autour de son axe central conjointement avec la révolution du récipient cylindrique autour d'un axe prédéfini de révolution ; et
obtention de la poudre lipidique séchée à partir du gel lamellaire lipidique par lyophilisation de la solution aqueuse malaxée de lipide.

13. Procédé selon la revendication 5, comprenant une étape où après (e), l'on ajoute de la bêta-propiolactone au fluide ou à la pâte obtenue après élimination de la substance fonctionnelle qui n'est pas encapsulée par le lipide.

14. Vésicule produite par le procédé selon l'une quelconque des revendications 1 à 13, ladite vésicule étant constituée d'un phospholipide de type phosphatidylcholine, d'un lipide chargé négativement, du cholestérol et d'un lipide lié au polyéthylène glycol, la température de transition de phase gel-liquide du phospholipide de type phosphatidylcholine étant inférieure à celle de la 1,2-dipalmitoyl-sn-glycéro-3-phosphatidylcholine.
